# EUROPEAN PATENT SPECIFICATION

(11) **EP 3 027 035 B1**
(45) Date of publication and mention of the grant of the patent: **11.04.2018**
(21) Application number: 14747612.1
(22) Date of filing: 31.07.2014
(51) Int. Cl.: A23C 9/12, A23C 9/123, C12N 1/20, G01N 33/569

(54) **GLUCOSE SECRETING LACTIC ACID BACTERIA STRAINS**
GLUCOSESEKRETIERENDE MILCHSÄUREBAKTERIENSTÄMME
SOUCHES DE BACTÉRIES LACTIQUES SÉCRÉTANT DU GLUCOSE

(30) Priority: 31.07.2013 NO 20131054
(43) Date of publication of application: 08.06.2016
(73) Proprietor: Tine SA, 0051 Oslo (NO)
(72) Inventor: KLINKENBERG, Geir, N-7072 Heimdal (NO); HOLO, Helge, N-1430 Ås (NO); ØYAAS, Jorun, N-7224 Melhus (NO)
(74) Representative: Onsagers AS
(86) International application number: PCT/EP2014/066502
(87) International publication number: WO 2015/014940

(56) References cited:
- WO-A1-2013/160413
- US-A- 5 071 763
- POOL W A ET AL: "Natural sweetening of food products by engineering Lactococcus lactis for glucose production", METABOLIC ENGINEERING, ACADEMIC PRESS, US, vol. 8, no. 5, 1 September 2006 (2006-09-01), pages 456-464, XP024946964, ISSN: 1096-7176, DOI: 10.1016/J.YMBEN.2006.05.003 [retrieved on 2006-09-01]
- VADEBONCOEUR C ET AL: "Control of sugar utilization in the oral bacteria Streptococcus salivarius and Streptococcus sanguis by the phosphoenolpyruvate: Glucose phosphotransferase system", ARCHIVES OF ORAL BIOLOGY, PERGAMON PRESS, OXFORD, GB, vol. 28, no. 2, 1 January 1983 (1983-01-01), pages 123-131, XP026164827, ISSN: 0003-9969, DOI: 10.1016/0003-9969(83)90119-X [retrieved on 1983-01-01]
- MONTVILLE T J ET AL: "Modified glucose-oxidase/peroxidase residual glucose assay for use with anaerobic bacteria", JOURNAL OF MICROBIOLOGICAL METHODS, ELSEVIER, AMSTERDAM, NL, vol. 6, no. 2, 1 January 1987 (1987-01-01) , pages 95-98, XP023699217, ISSN: 0167-7012, DOI: 10.1016/0167-7012(87)90057-1 [retrieved on 1987-01-01]
- P. T. C. VAN DEN BOGAARD ET AL: "Control of Lactose Transport, beta -Galactosidase Activity, and Glycolysis by CcpA in Streptococcus thermophilus: Evidence for Carbon Catabolite Repression by a Non-Phosphoenolpyruvate-Dependent Phosphotransferase System Sugar", JOURNAL OF BACTERIOLOGY, vol. 182, no. 21, 1 November 2000 (2000-11-01), pages 5982-5989, XP055147276, ISSN: 0021-9193, DOI: 10.1128/JB.182.21.5982-5989.2000

## Description

### Field of the invention

The present invention relates to a method for obtaining glucose secreting lactic acid bacteria strains useful for the preparation of fermented dairy products, such as e.g. yogurt, and to lactic acid bacteria strains obtained by said method.

### Background of the invention

Sugar became the principle sweetener in Europe and North America in the 17^{th} century along with the increasing production of sugar in Brazil and West Indies colonies resulting in that sugar was no longer a luxury product for the prosperous population only. Since then, and along with the industrialization and increased providing of industrial processed foods and various sweetened beverages, sugar consumption has steadily increased in the population.

With the increasing consumption of sugar, an increasing awareness of the possible adverse effects have arisen with the health and nutrition authorities, e.g. in respect of the expected risk of developing diabetes and obesity in the population. The expected negative effect of a high sugar diet in the population results in that the health and nutrition authorities aim at reducing the consumption of sugar, and demands for the development of novel food products with less added sugar.

Various bacteria are commonly used in the food industry, e.g. in order to improve taste, texture and/or shelf life of products. Within the dairy industry, various lactic acid bacteria have been used as "starter cultures" in dairy fermentation, e.g. in manufacturing of yogurt, cheese and fermented milk products. The starter cultures are added to the milk to be fermented and are allowed to grow under controlled conditions, resulting in the production of lactic acid and modification of the flavor and texture of the fermented product.

Yogurt as well as other fermented dairy products on the marked, contains sugar added in order to provide the desired sweet taste required by the consumers. The amount of added sugar varies depending on the addition of other ingredients, such as e.g. the type and amount of fruits and/or berries. Addition of other (artificial) sweeteners may replace the added sugar, but generally results in a less attractive product from a consumer point of view due to the increasing demand for food products being free of artificial additives.

The lactic acid bacteria utilized in the production of fermented dairy products grow on the lactose present in the milk. In *Streptococcus thermophilus* and *Lactobacillus bulgaricus,* lactose is taken up by a lactose transporter, then split to glucose and galactose and the latter is secreted by the lactose transporter in exchange with incoming lactose.

An insignificant amount of the lactose is also transformed to various other polysaccharides and aromatic compounds which nevertheless are of importance for obtaining the desired taste and texture of the fermented product.

The lactic acid bacteria used in the manufacturing of fermented dairy products available on the marked metabolize only the lactose needed for the growth of the microorganisms used until a pH of about 4.5 is reached. In general, milk comprises about 48 g/l lactose prior to fermentation. However, since the lactic acid bacteria only metabolize the amount of lactose needed for self consumption, far from all of the lactose present in milk is metabolized by the lactic acid bacteria known in the prior art. For example, when fermenting milk in production of yoghurt, lactic acid bacteria metabolize about 16% of the lactose available in the milk. The remaining lactose is not cleaved.

As the sweetness of lactose is minor compared with the sweetness of galactose, and importantly, glucose, the lactose remaining in the fermented milk at the end of the fermentation process constitute an unexploited source of sweetness.

Of the about 48 g/l lactose contained in the milk prior to the fermentation process, about 8 g/l of the lactose is at maximum metabolized to lactic acid until the fermentation process ends when pH of about 4.5 is reached. The amount of galactose available is therefore at the most about 4.0 g/l. Thus, if all the galactose produced by the cleavage of the 8 g/l lactose is accumulated, this would only result in an insignificant reduction of the sugar addition needed in order to obtain a fermented product with acceptable sweetness.

Besides, even though all the galactose available in the lactose in the milk is released by cleavage of essentially all the lactose, the increase in sweetness due to increased secretion of galactose would not alone result in a substantial reduction in the need for additional sugar.

However, in case the potential of sweet taste of the glucose inherent in the unexploited lactose of milk could be utilized, e.g. by providing lactic acid bacteria with increased lactose hydrolysis and glucose secretion characteristics, fermented diary products with a significant less need for added sugar may be obtained.

Pool et al used genetic engineering in construction of a mutant of *Lactococcus lactis* unable to degrade glucose and that secreted glucose when grown on lactose (Pool et al., 2006, " Natural sweetening of food products by engineering Lactococcus lactis for glucose production", Metab Eng 8:456-464.). The amount of sugars in the supernatants of the *L. lactis* mutant was determined by HPLC.

Van den Bogaard et al., reported in Journal of Bacteriology, 2000, Vol. 182, No. 21, pp. 5982-5989 about a ccpA disruption *Streptococcus thermophilus* strain found to release glucose into the medium when grown on lactose. However, the primary effects of the ccpA disruption where prolonged lag time and reduced growth rate on all sugars tested.

Ibrahim and O'Sullivan report the use of chemical mutagenesis for the isolation of food grade β-galactosidase overproducing mutants of *Bifodobacteria*, *Lactobacilli* and *Streptococcus thermophilus*, identified using the o-nitrophenyl- β -galactoside assay (Ibrahim and O'Sullivan, 2000, "Use of chemical mutagenesis for the isolation of food grade beta-galactosidase overproducing mutants of Bifidobacteria, Lactobacilli and Streptococcus thermophilus", Jour. Dairy Sci., 83, pp. 923-930). The aim of the protocol reported therein is the providing of probiotic cultures with increased β-galactosidase concentrations useful in the treatment of symptoms of lactose malabsorption in humans.

Thompson et al., 1985, 162:1, 217-223 relates to a Streptococcus lactis 133 strain lacking glucokinase and mannose phosphotransferase activities. The double mutant *S. lactis* 133 mannose-PTS^{d} GK⁻ disclosed therein is unable to utilize either exogenously supplied or intracellularly generated glucose for growth.

US 5,071,763 disclose the providing of one mutant of *S. thermophiles* strain having a defective lactose transport system stated to be useful in the preparation of lactose reduced or lactose free dairy foods.

Various methods for screening microorganisms are known, also methods that provides for automated culturing and screening of microorganisms involving several different and repetitive functions and tasks, such as e.g. culturing facilities for the strains to be screened, inoculation of liquid culture, centrifugation, pipetting, dilution, transfer of samples, assaying facilities and operations, and suitable hardware, robots and computers allowing for the handing and manipulation of a large number of samples at the same time. Such systems provides for high-throughput screening of numerous microorganism isolates, e.g. using microtiter plates adapted for the screening of from 6 to above 1500 samples of microorganisms at the same time (e.g. GigaMatrix® Ultra High-Throughput Screening platform).

WO 01/32844 disclose a microtiter plate based high throughput screening assay for the screening of a large number of cell populations producing variants of a molecule of interest, such as e.g. α-amylase, and wherein samples from the wells of the microtiter plate is assayed e.g. by detection of e.g. fluorescence, luminescence, enzyme activity etc..

Even though various automated systems for screening microorganisms are available, the use thereof depends on whether assays for analyzing the desired characteristics of the microorganism tested are available, and also whether such assays are easily automated and suitable for large scale screening systems. The methods commonly used in order to detect and screen for lactic acid bacteria useful in the dairy industry today, are to our knowledge in essence based on methods dependent on the use of HPLC or based on the measuring of the activity of a particular enzyme of interest, such as the activity of lactase or glucokinase, growth rate etc.. From an industrial point of view, the use of HPLC in order to determine the ability of a lactic acid bacteria to metabolize a nutrient, or to produce a desired metabolite, is not desirable as it is time consuming and inefficient. Although the use of various enzymatic methods for determining glucose in a sample of a lactic acid bacteria culture have been described, cf. e.g. Thomson et al. above, such methods have to our knowledge not been utilized in large scale screening methods. Furthermore, as shown below (example 3), high throughput screening based on a well known enzyme activity assay were found to be inaccurate and inefficient.
Fermented dairy products with less or no sugar added may be provided by the use of a lactic acid bacterium strain that have the ability to hydrolyze more of the unexploited lactose present in the milk, and to secrete the resulting glucose into the fermented product. In order to efficiently screen for glucose secreting lactic acid bacteria mutants, there is however a need for developing methods enabling the identification of such mutants which do not depend on bothersome and time consuming assaying. Thus, it is an object of the present invention to provide efficient automated screening methods for identifying lactic acid bacteria with the ability to secrete glucose.

### Summary of the Invention.

The object of the present invention is obtained by the providing of a mutagenesis and large scale screening method where the identification of glucose secreting mutants is performed utilizing enzymatic assaying. More particular, the large scale screening method of the present invention comprises the steps of:
i) subjecting lactic acid bacteria to mutagenizing conditions, wherein the mutagenizing condition consists in exposure of lactic acid bacteria to any conventional mutagens of physical (e.g. X-radiation, UV-radiation), or chemical (e.g. mutagens) origin;ii) selecting the colonies obtained in step
   i) separately and transfer said colonies for growth in an array in growth media comprising lactose as the sole carbohydrate source;
   iii) assaying the amount of glucose present in the culture supernatant after growth of step ii) by the use of:
      a) glucose oxidase; peroxidase; and peroxidase signal substrate; or
      b) glucokinase, hexokinase and glucose-6-phosphate dehydrogenase, ATP and NAD or NADP; or:
      c) glucose dehydrogenase and an electron acceptor; wherein the amount of glucose secreted into the culture medium is proportional with the amount of signal substance obtained by the transformation of the peroxidase signal substrate in a) or the amount of NADH or NADPH produced in b), or the amount of reduced electron acceptor in c).

Despite the fact that prior art shows that fumbling with the lactose and glucose transport and metabolism of lactic acid bacteria may result in negative effects (such as e.g. poor growth characteristics), the present inventors provides a lactic acid bacterium strain having ability to take up and cleave more lactose than needed for the growth of the bacteria, and which furthermore secrete more glucose into the fermentation media compared with the corresponding wild type strain. According to one embodiment, the lactic acid strains subjected to the method of the present invention are sonicated after and optionally prior to step i).

According to another embodiment of the present method, the colonies obtained in step i) is separately selected and transferred for growth in medium comprising lactose as the sole carbohydrate source in an array.

According yet another embodiment, the parenteral lactic acid strain is selected from the group consisting of strains of *Lactococcus spp, Streptococcus spp.* (e.g. *Streptococcus thermophilus)*, *Lactobacillus spp.* (e.g. *Lactobacillus bulgaricus, Lactobacillus acidophilus*, *Lactobacillus subsp. casei)*, *Leuconostoc spp., Pseudoleuconostoc spp, Pediococcus spp., Brevibacterium spp., Enterococcus spp*, *Propionibacteriusm spp.,* and *Bifidobacteriusm spp,* preferably *S. thermophilus.*

According yet another embodiment of the present invention, the mutagenizing condition in step ii) consist in culturing the lactic acid bacteria in the presence of a mutagen, such as e.g. a mutagen selected from the group consisting of n-methyl-N'-nitro-N-nitroguanidine (NTG), and ethane methanesulphonate (EMS).

According a further embodiment of the present method, the screening of step (iv) is performed using glucose oxidase, peroxidase and a peroxidase signal substrate. The said peroxidase substrate may according to one embodiment be selected from the group consisting of 4-amino-antipyrene, 4-amino-antipyrine, 2,2'-azino-bis(3-ethylbenzthiazoline-6-sulphonic acid) (ABTS), o-dianisidine, o-phenylenediamine (OPD), and 4-(2-pyridylazo)resorcinol, N-acetyl-3,7-dihydroxyphenoxazine (Amplex Red), preferably 4-amino-antipyrine.

According to yet another embodiment of the present invention, the amount of signal substance or NAHD or NADPH or reduced electron acceptor formed in iv) is measured photometrically, e.g. measured by spectrophotometry, luminescence or fluorescence.

According to yet another embodiment of the present invention, a large scale screening method is provided for identifying a glucose secreting *S. thermophiles* strain, wherein the process comprises the steps of:
i) subjecting *S*. *thermophiles* bacteria to mutagenizing conditions, wherein the mutagenizing condition consists in exposure of lactic acid bacteria to any conventional mutagens of physical (e.g. X-radiation, UV-radiation), or chemical (e.g. mutagens) origin;
ii) selecting the colonies obtained in step i) separately and transfer said colonies for growth in an array in growth media comprising lactose as the sole carbohydrate source;
iii) assaying the amount of glucose present in the culture supernatants after growth of step iii) by the use of glucose oxidase; peroxidase; and a peroxidase signal substrate;
wherein the amount of glucose secreted into the culture medium is proportional with the amount of signal substance obtained by the transformation of the peroxidase signal substrate in iii).

According to yet another embodiment of the present invention, a glucose secreting bacterial strain is provided, wherein said strain is selected from the group consisting of:
a) the lactic acid 2-83 strain deposited under the Budapest Treaty at DSMZ with accession number DSM 27535 or the lactic acid strain 10.44 deposited under the Budapest Treaty at DSMZ with accession number DSM 29077, and related strains having substantially the same genotypic and phenotypic characteristics in respect of lactose transport, lactose hydrolysis and/or glucose secretion; and
b) a lactic acid bacterial strain which is a mutant of the strain of a), having the ability to secrete at least 8 g/l glucose into the fermented product.

According to yet another embodiment of the present invention, a glucose secreting lactic acid bacterial strain is provided, wherein said strain is selected from the group consisting of:
a) the lactic acid 2-83 strain deposited under the Budapest Treaty at DSMZ with accession number DSM 27535 or the lactic acid strain 10.44 deposited under the Budapest Treaty at DSMZ with accession number DSM 29077, and related strains having substantially the same genotypic and phenotypic characteristics in respect of lactose transport, lactose hydrolysis and/or glucose secretion; and
b) a lactic acid bacterial strain which is a mutant of the strain of a), having the ability to secrete at least 9 g/l glucose into the fermented product.

According to yet another embodiment of the present invention, a glucose secreting bacterial strain is provided, wherein said strain is selected from the group consisting of:
a) the lactic acid 2-83 strain deposited under the Budapest Treaty at DSMZ with accession number DSM 27535 or the lactic acid strain 10.44 deposited under the Budapest Treaty at DSMZ with accession number DSM 29077, and related strains having substantially the same genotypic and phenotypic characteristics in respect of lactose transport, lactose hydrolysis and/or glucose secretion; and
b) a lactic acid bacterial strain which is a mutant of the strain of a), having the ability to secrete at least 10 g/l glucose into the fermented product.

The present invention specifically provides *S*. *thermophilus* strains deposited at DSMZ (Leibniz-Institute DSMZ-Deutsche Sammlung von Mikroorganismen und Zellkulturen GmbH) under the accessions numbers DSM 27535 and DSM 29077, respectively, and also glucose secreting lactic acid bacterial strains obtained by the method according to the present invention.

The present lactic acid bacterium strain according to the present invention may be used in the manufacturing of a fermented milk product. The present invention thus provides various fermented dairy product comprising a glucose secreting bacterial strain identified by the method according to the present invention.

Finally, a further embodiment of the present invention is the use of glucose oxidase, peroxidase, glucokinase, hexokinase, glucose-6-phosphate dehydrogenase and glucose dehydrogenase in a high through put screening method for identification of glucose secreting lactic acid bacteria.

### Brief description of the figures

Figure 1: shows the growth curve of a strain of *S*. *thermophilus* isolated from the commercial available culture YF-L702.
Figure 2: shows the results of enzymatic assay of the supernatant of mutants of a strain of *S*. *thermophilus* isolated from the commercially available culture YF-L702. The cultures were screened in 4 parallel wells, and the average concentration of glucose was calculated. The standard deviation is given for all the measured samples.
Figure 3: shows the pH in milk cultures during fermentation at 42 °C by *S*. *thermophilus* and *Lb. delbruckii subsp. Bulgaricus,* wherein the solid line represent mutant strains and wherein the dashed line represents wild type strains.

### Detailed description of the invention

The present invention provides a high throughput screening method for identifying glucose secreting lactic acid bacteria strains based on the findings that the level of glucose secreted into the medium of lactic acid bacteria being subjected to mutagenesis could efficiently and automatically be measured utilizing an enzyme based assay.

According to the present invention, the expression "mutagenizing conditions" is meant to comprise any method suitable for the introduction of random mutations in the genome of one or more microorganisms, such as lactic acid bacteria strain(s). For example, mutagenizing conditions may be the exposure of lactic acid bacteria to any conventional mutagens of physical (e.g. X-radiation, UV-radiation), or chemical (e.g. mutagens) origin.

Numerous chemical mutagens may be used according to the present invention, such as e.g. mutagens selected from the group consisting of n-methyl-N'-nitro-N-nitroguanidine (NTG), and ethane methane sulphonate (EMS).

According to one aspect of the present invention, ethane methane sulphonate (EMS) is preferably used.

In order to provide an efficient method enabling high throughput screening of lactic acid bacteria according to the present invention, it is vital that the assaying method is sensitive and reliable. As an increased ability to secrete more glucose may be due to an increased lactase activity, the present inventors measured the glucose secreting capacity of mutated strains by measuring the effects of growing the mutagenized strains in the presence of 5-bromo-4-chloro-indolyl-β-D-galactopyranoside (X-Gal, CAS No. 7240-90-6). X-Gal is a chromogenic substrate that produces a rich blue color when cleaved by lactase. The blue color formed can easily be detected visually over background and measured using a spectrophotometry. Others have previously screened for lactase overproducing *S*. *thermophilus* strains by culturing the mutants to be tested on agar comprising X-gal (Ibrahim and O'Sullivan (2000), *supra).* However, as seen in example 3, this method was not applicable in a high throughput screening method for determination of glucose secreting lactic acid bacteria.

The present inventors therefore had to develop other methods useful for the purpose of identifying glucose secreting lactic acid bacteria. Instead of measuring lactase activity of the obtained mutants, the inventors aimed at finding a method enabling the direct measuring of glucose concentration, and without using HPLC.

Glucose concentration is measured in biological samples, such as serum, blood, or urine, utilizing various commonly available glucose assays, where glucose contained in the sample to be analyzed is oxidized through the addition of glucose oxidase, resulting in the formation of D-glucono-γ-lactone and hydrogen peroxide (H₂O₂). The H₂O₂ is further reacted with a signal forming substrate in the presence of yet a second enzyme, peroxidase, forming a signal compound which may be measured photometrically. The term "assaying the amount of glucose by the use of glucose oxidase; peroxidase; and a peroxidase signal substrate" as used in the present application refers to the use of a system as described above, wherein the addition of glucose oxidase results in the oxidation of glucose to produce hydrogen peroxide and D-glucono-δ-lactone. The production of hydrogen peroxide in this system is then in turn involved in the transformation of a signal forming substrate to a signal forming compound due to the presence of the peroxidase. The amount of the glucose present in the culturing medium is thus proportional with the amount of signal compound formed.

This principle have been used e.g. to analyze microorganism with increased glucose oxidase activity, see Fiedurek et al. (1986), Enzyme Microbiol. Technol, vol 8, pp 734 - 736. A glucose-oxidase/peroxidase assay has also been used in order to detect residual glucose in culture medium of lactic acid bacteria, i.e. thus studying the glucose consumption, in some Lactobacillus species (Montville and Hsu (1987), Journal of Microbiol. Methods, 6, pp. 95-98). Montville and Hsu indicated that anomalously high glucose values in the glucose oxidase/peroxidase assay was caused by the presence of endogenous peroxide, and suggest the need for preincubation with catalase.

The use of glucose oxidase, or any other enzyme systems useful in order to measure the amount of glucose in a sample, has not previously been used in a large scale screening of numerous bacteria.

According to the present invention, the screening for mutants having the desired ability to secrete glucose into the dairy product to be fermented is provided utilizing an enzyme based automated screening method directly measuring the amount of glucose secreted by the tested strains. The enzyme based method is found to be superior compared with the lactase activity/X-gal assay. Contrary to the teaching of the prior art, the present inventor furthermore did not find it necessary to add any catalase in order to obtain reliable results when screening *S*. *thermophilus* mutants (cf. Montville and Hsu, *supra*).

Other enzymatic systems may also be used to measure the amount of glucose secreted by lactic acid bacteria subjected to the present mutagenesis method, for example, glucokinase, hexokinase and glucose-6-phosphate dehydrogenase, glucose dehydrogenase, together with ATP, and NAD or NADP or other suitable electron acceptors as substrates, respectively.

If utilizing hexokinase or glucokinase for determination of glucose concentration, hexokinase, glucose-6-phosphate dehydrogenase, ATP and NAD or NADP is added to the solution expected to comprise glucose. Hexokinase reacts with D-glucose to form glucose-6-phosphate in the presence of ATP. Glucose-6-phosphate dehydrogenase is further acting upon the obtained glucose-6-phosphate in the presence of NAD or NADP forming NADH or NADPH, respectively.

The amount of NADH or NADPH may be measured using well known spectrophotometry methods. The amount of NADPH or NADH is proportional with the amount of glucose present in the sample.

In yet an alternative enzyme based screening method, glucose dehydrogenase may used, which in the presence of an electron acceptor transform D-glucose to D-glucono-1,5-lactone and reduce the electron acceptor. The amount of reduced electron acceptor may then be measured by any suitable spectrophotometry method. Various useful electron acceptors may be used, such as e.g. NAD and NADP.

The present method provides for a high throughput screening of glucose secreting lactic acid bacteria, i.e. where handing of liquid, clones, compounds or substrates to be added etc. may be performed by automated fluid handling systems and robotics. For example, samples of the cultures of the mutagenized lactic acid bacteria strains may be assayed utilizing an automated handling system with an integrated handling robot enabling the isolation of mutants and transfer of the mutagenized lactic acid bacteria to microtiter plates for further handling.

Prior to the selection of clones to be tested, the mutagenized lactic acid bacteria strain is transferred to growth medium comprising lactose as the sole carbohydrate source. An excess of lactose is used in order to mimic the growth conditions present in milk fermentation.

The separately selection and isolation of clones may also be performed using suitable cell sorting flow cytometers well known to the skilled person. Clones to be screened according to step iii) of the present method may be selected and transferred to microtiter plates by the use of any suitable colony picking robot and cultured. Samples of the culture media obtained, optionally the supernatants obtained after centrifugation of the cultured strains, are then subjected to the automated enzyme assay Before and after the lactic acid strains are exposed to mutagenizing conditions, the culture are preferably sonicated in order to separate the individual cells.

The selection and transfer of colonies may be performed using a colony picking robot, such as a Genetix Q-pixII robot (Genetix colony pickers, Molecular Devices). The handling of the cultured strains, and the culture medium thereof may be performed using a liquid handling robot, such as e.g. Beckman Coulter liquid handling system with an integrated Beckman Coulter NXP liquid handling robot (Beckman Coulter, Inc.). Other automated liquid handling systems and colony picking robots may also be used.

According to one embodiment, a liquid handling robot is used to transfer samples of the supernatants to suitable microtiter plate wells comprising glucose oxidase, peroxidase and a peroxidase signal forming substrate. The mentioned enzymes and substrate may optionally be added to the supernatants. Upon oxidation of the glucose present in the supernatants by the glucose oxidase, H₂O₂ is produced. Peroxidase catalyzes a reaction of H₂O₂ and the peroxidase signal forming substrate resulting in the formation of a signal compound. The amount of signal compound formed is proportional with the amount of glucose present in the sample. Based on a suitable standard curve adapted to the glucose/peroxidase/peroxidase substrate used, the amount of glucose is determined photometrically, e.g. by spectrophotometry, fluorescence, luminescence or any other suitable photometric method.

Various peroxidase signal forming substrates may be used according to the present invention, such as e.g. a substrate selected from the non-limiting group of 4-amino-antipyrene, 4-amino-antipyrine, 2,2'-azino-bis(3-ethylbenzthiazoline-6-sulphonic acid) (ABTS), o-dianisidine, o-phenylenediamine (OPD), 4-(2-pyridylazo)resorcinol, and N-acetyl-3,7-dihydroxyphenoxazine (Amplex Red).

According to one embodiment of the present invention, the peroxidase substrate is 4-amino-antipyrine. Lactic acid bacteria is as mentioned commonly used in the dairy industry and covers in general organisms that produce lactic acid as the principle by-product of the fermentation process. Lactic acid bacteria is in the dairy industry most often used as "starter cultures", i.e. added to milk and grown under controlled conditions in order to produce lactic acid, reduce the pH and produce the desired flavor and texture of the fermented product.

The term "lactic acid bacteria" as used in the context of the present invention, is to be understood to mean any lactic acid bacteria useful in the manufacturing of fermented dairy products upon fermentation of milk comprising lactose. For example, lactic acid bacteria that may be subjected to the method of the present invention may be selected from the non-limiting group consisting of *Lactococcus spp, Streptococcus spp. (*e.g. *Streptococcus thermophilus), Lactobacillus spp. (*e.g. *Lactobacillus bulgaricus, Lactobacillus acidophilus*, *Lactobacillus subsp. casei)*, *Leucohostoc spp., Pseudoleuconostoc spp, Pediococcus spp., Brevibacterium spp., Enterococcus spp, Propionibacteriusm spp.,* and *Bifidobacteriusm spp..*

Lactic acid bacteria used in the dairy industry are commonly supplied and available to the dairy industry as frozen or freeze dried cultures. Any lactic acid bacteria used in dairy fermentation processes may be subjected to the mutagenesis and screening method according to the present invention. Various lactic acid bacteria that may be used in accordance with the present method are commercially available, e.g. from DSMZ (Leibniz Institute DSMZ - German Collection of Microorganisms and Cell Cultures, http://www.dsmz.de/), from the microbiology collection of ATCC (http://www.lgcstandards-atcc.org/en/Products/Collections/Microbiology_Collections.aspx), NCIMB (The national collection of Industrial, Marine, and Food Bacteria, http:///www.ncimb.com/culture.html), or from other commercial suppliers such as e.g. Chr. Hansen AS (http://www.chr-hansen.com/products/product-areas/dairy-cultures.html). Furthermore, lactic acid bacteria strains that may also be isolated from yoghurt, cheese, cultured milk, and fermented food in general.

Any lactic acid bacteria as mentioned above may be subjected to the method according to the present invention. According to one embodiment, the lactic acid bacteria subjected to the method of the invention is *S. thermophilus.*

As used herein a "glucose secreting lactic acid bacterium or bacteria" or "lactic acid bacteria mutant or mutants" refer to a lactic acid bacterium strain having the ability to hydrolyze a substantially amount of the lactose present in a diary product to be fermented, and wherein there are less need for added sugar in a fermented dairy product produced with the glucose secreting lactic acid bacterium of the present invention compared with similar products prepared using the parenteral/wild type strain.

The present method furthermore provides for a method to identify lactic acid mutant strains having the ability to secrete a substantial amount of glucose into the fermented dairy product.

Without being bound by theory, it is believed that the lactic acid bacterium strain of the present invention is capable of increased lactose uptake, increased lactase activity and increased glucose secretion.

According to the present invention, "a substantial amount of lactose" is to be understood to mean that the lactic acid bacterium of the present invention is capable of hydrolyzing more lactose than the corresponding wild type/parental strain or strains of the same species. For example, according to one embodiment of the invention, the lactic acid bacterium of the present invention is capable of hydrolyzing at least about 50 % of the lactose present in the milk to be fermented, such as at least about 60%, such as at least about 70%, such as at least about 80%, such as at least about 90%, or such as at least about 95%. According to another embodiment of the present invention, the glucose secreting lactic acid bacterium of the present invention is capable of hydrolyzing essentially all the lactose present in the milk to be fermented.

According to the present invention, "a substantial amount of glucose" is to be understood to mean that the glucose secreting lactic acid bacterium of the present invention is capable of secreting more glucose into the fermented dairy product than the corresponding wild type/parental strain or strains of the same species not being subjected to the method according to the present invention.

For example, the glucose secreting lactic acid bacteria of the present invention is according to one embodiment capable of secreting at least about 8 g/l glucose into the fermented dairy product, such as at least 9 g/l, glucose 10 g/l glucose, such as at least about 15 g/l glucose, such as at least 20 g/l glucose, such as at least about 30 g/l glucose, such as at least about 40 g/l glucose.

The glucose secreting lactic acid bacteria of the present invention may be used alone or together with other lactic acid bacteria strains well known to the skilled person. For example, in case the lactic acid bacteria are *S*. *thermophilus*, it may be used together with *Lactobacillus delbrueckii subsp. bulgaricus.*

According to the present invention, a glucose secreting bacterial strain is provided selected from the group consisting of:
a) the lactic acid 2-83 strain deposited under the Budapest Treaty at DSMZ with the accession number DSM27535 or the lactic acid strain 10.44 deposited at DSMZ with the accession number 29077, and related strains having substantially the same genotypic and phenotypic characteristics in respect of lactose transport, lactose hydrolysis and/or glucose secretion; and
b) a lactic acid bacterial strain which is a mutant of the strain of a), having the ability to secrete at least 8 g/l glucose into the fermented product.

According to one embodiment, the present invention relates to the novel glucose secreting strains 2-83 deposited under the Budapest Treaty at DSMZ the 12^{th} July 2013 with the accession number DSM27535 and the strain 10.44 deposited under the Budapest Treaty at DSMZ the 11^{th} July 2014 with the accession number DSM29077, which are both isolated according to the large scale screening method of the present invention.

It is to be understood that the present disclosure covers lactic acid bacterial strains having substantially the same glucose secreting ability as the strains identified according to the present invention, such as e.g. the deposited strain mentioned above and thus having substantially the same genotypic and phenotypic characteristics in respect of lactose transport, lactose hydrolysis and glucose secretion. The skilled person will acknowledge that various substitutions, deletions or additions to the genomic sequence may be introduced by commonly available gene technology methods or randomly during culturing of a lactic acid bacterium strain, and that such alteration may be introduced with purpose or randomly/accidentally without substantially changing the characteristics of the bacterial strain. It is to be understood that glucose secreting lactic acid strains differing from the strains of the present invention merely by the purposely or randomly introduction of such alteration in the genome that do not substantially effect the lactose transport, lactose hydrolysis and/or glucose secretion abilities are covered by the present disclosure The expression "related strains having substantially the same genotypic characteristics" is thus to be understood to mean glucose secreting strains where the relevant parts of the genome of the strains to be compared has a high degree of sequence identity compared with a lactic acid bacteria of the same species not having the ability to secrete glucose in the fermented dairy product. It is furthermore to be understood that "related strains having substantially the same genotypic characteristics" in the context of the present invention means that the strain of question has at least substantially the same lactose transport, lactose hydrolyzing, and/or glucose secretion ability as a lactic acid bacterial strain of the present invention, such as e.g. the strain 2-83 (DSM27535) and the strain 10.44 (DSM29077), e.g. as identified according to the screening method of the present invention.

The expression "related strains having substantially the same phenotypic characteristics" as used herein is to be understood to refer to one or more observable properties of lactic acid bacteria that are the result of the interaction of the inherent genotype of the lactic acid bacteria and/or the non-hereditary environmental variations. In respect of the present invention, the term "substantially the same phenotypic characteristics" is to be understood to mean the characteristics of having substantially the same lactose transport, lactose hydrolyzing, and/or glucose secretion ability as the lactic acid bacterial strain of the present invention, such as e.g. the strains DSM27535 and DSM29077, e.g. as identified according to the screening method of the present invention.

It is furthermore to be understood that a related strain having substantially the same genotypic or phenotypic characteristics means a strain that upon screening according to the present invention, exert the ability to secret glucose in an amount that render it possible to reduce or remove the need for additional sugar when said strain is used in the fermentation of milk.

It is furthermore to be understood that also further mutants originating from a glucose secreting strain obtained by the present method, i.e. such as a functionally equivalent mutant having substantially the same lactose transport, lactose hydrolyzing, and glucose secreting abilities, is covered by the present invention.

The term "mutant thereof" as used herein is thus to be understood to mean a strain derived from a strain of the present invention by means of e.g. genetic engineering, mutagenizing conditions or by culturing.

According to yet another embodiment, the glucose secreting lactic acid bacteria of the present invention is a lactic acid bacterium strain identified using the screening method of the present invention.

The mutated lactic acid bacteria obtained according to the present invention may be used to prepare various fermented dairy products. As used herein, "fermented dairy product" refers to products intended for animal consumption, more specifically for human consumption, and which are derived by fermentation and by the use of a lactic acid bacterium according to the present invention. As a non-limiting example, "fermented dairy product" refers to product selected from the group consisting of yogurt, cheese and fermented milk products.

It is furthermore to be understood that such fermented dairy products may in addition comprise secondary ingredients, such as fruit, berries, flavors, etc. It is furthermore to be understood that "fermented dairy products" according to the present invention comprise less added glucose as sweetener compared with fermented dairy products prepared by prior art lactic acid bacterium which do not have the increased glucose secretion ability as the glucose secreting strains of the present invention.

It is to be understood that the *S. thermophilus* strains according to the present invention may be used in the fermentation of milk in combination with other bacterial strains commonly used in fermentation of milk. For example, the *S*. *thermophilus* strains may be used together with one or more *Lactobacillus delbruckii spp. bulgaricus,* such as together with the *Lactobacillus delbruckii spp. bulgaricus* strain 9A deposited the 11^{th} July 2014 at DSMZ with the accession number DSM29076.

A composition is furthermore provided comprising a suitable amount of one or more *S. thermophilus* strain according to the present invention, and one or more *Lactobacillus delbruckii spp. bulgaricus* strain(s).

According to one embodiment, a composition is provided comprising S.thermophilus strain 10.44 (DSM29077), and *Lactobacillus delbruckii spp. bulgaricus* strain 9A (DSM29076).

### Examples

For illustration purposes, the following examples are given. The terminology of the present specification is to be interpreted by the skilled person in light of the teachings and guidance presented herein, in combination with the general knowledge of the skilled person.

### Example 1: Isolation, culturing and characterization of S. thermophilus strains

Strains of *S. thermophilus* were isolated from *S*. *thermophilus* starter cultures (commercially available from Chr. Hansen) by streaking on M17 agar plate supplemented with 5 g/l galactose. The agar plates were incubated at 42 °C over night. from the agar plate were inoculated in liquid M17 comprising 5 g/l galactose and incubated over night. Samples of the obtained cultures were added glycerol and stored at -80 °C.

The culture broth M17 Oxoid CM0817 (Oxoid Microbiology Products by Thermo Scientific) was stored and prepared according to the prescription of the manufacturer. M17 agar plates comprised 15 g agar/l.

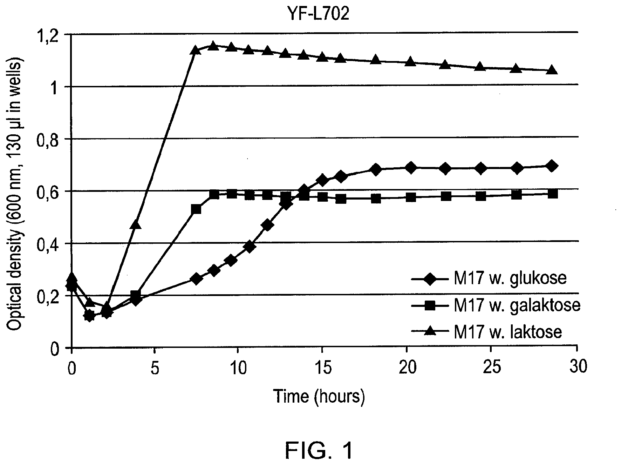

In order to investigate the growth kinetics of the cultured *S. thermophilus,* the isolated strains were grown in microtiter plates in the presence of glucose, galactose and lactose, respectively, in different concentrations (5, 24 and 48 g/l, respectively). 3 vol% of over night cultures cultured in M17 comprising the same carbon source were added to each well and incubated at 42°C (humid atmosphere) in a Cytomat 2 450A incubator integrated in a robotized liquid handling system. Optical density was measured at 600 nm in the cultures each hour for about 28 hr. The growth curve for the tested YF-L702 culture is shown in figure 1.

### Example 2: Mutagenesis of S. thermophilus

Three strains of *S. thermophilus* obtained in example 1 were grown until early stationary phase (50 ml tubes, in M17 at 37°C and with rotation). A sample (20 ml) of each strain was transferred to another tube and sonicated (output 5, duty cycle 40%, 20 sec, Branson Sonifier 250). Each culture were diluted with preheated M17 and cultured for 2 hr with rotation. Samples of each culture was then exposed to ethyl methane sulfonate (EMS) at various concentrations (0, 0.25, 0.5, 1.0, and 2.0 %v/v) and incubated for 60 min at 37°C with rotation. The samples were then centrifuged, washed with ice cold 0.9/ NaCl, and re-suspended in 12 ml M17. Prior to further culturing, storage or analysis, the samples were again sonicated.

### Example 3: Screening for glucose secreting mutants based on lactase activity assay.

Mutants subjected to the mutagenesis method in example 2 were initially tested for their ability to produce glucose by measuring the lactase activity based on the tested mutant's ability to cleave the substrate 5-bromo-4-chloro-indolyl-β-D-galactopyranoside (X-gal). The cleavage of X-gal results in the development of a strong, blue color in the presence of lactase. Said method have previously been used to identify lactase over expressing mutants of *S*. *thermophilus* cultured on BHI agar (Brain Heart infusion, Oxoid CM1135) comprising X-gal (Ibrahim and O'Sullivan (2000), *supra).*

Initially, a few samples of strains exposed to EMS were transferred to M17 agar comprising lactose and glucose, or BHI agar comprising glucose, and added 10 µglml and 40 µg/ml X-Gal, respectively. The results showed that the tested strains developed blue color over time irrespective of the amount of X-gal added. This was unexpected as Ibrahim and O'Sullivan demonstrated that colonies of wild type strains of *S*. *thermophilus* were approx. white on BHG-agar comprising 40 µg /ml X-gal. Some minor difference was observed in time and intensity of the color development although the colonies became blue irrespective of the X-gal concentrations.

The screening method was then performed on a larger population of *S*. *thermophilus* mutants. About 20 000 colonies from *S*. *thermophilus* were cultured in the presence of 1% EMS in accordance to the method of example 2 were plated on 10 M17 agar plates (25 x 25 cm) comprising 5 mg/ml glucose and 15 µg/ml X-gal. 7 colonies which appeared to have a better developed blue color was screened in liquid M17 medium comprising 48 and 254 g/ml lactose, respectively, to determine lactose metabolism. At least two of the isolated mutants showed some glucose accumulation in the medium during culturing. No significant accumulation of glucose in the media was measured in the media of the wild type strain of *S*. *thermophilus* although colonies of the wild type strain developed blue color.

Although mutants accumulating glucose in the media could be determined by the above method based on lactase activity, the screening of *S. thermophilus* strains based on their over expression of lactase and the development of blue color when cultured in the presence of X-gal was found to be difficult, labor-intensive and inefficient as the wild type strains of *S. thermophilus* also developed blue color. In addition, the degree of color development varied dependent on the size and density of the colonies.

### Example 4: Screening of glucose secreting mutants based on determination of glucose concentration in the culture medium.

*S. thermophilus* strains obtained in example 1 were cultured in M17 medium in the presence of EMS according to example 2 using a culture robotic liquid handling system with an integrated incubator and integrated liquid handling robot (Beckman Coulter core robot system).

A mutant library was obtained by plating the mutagenized samples of bacteria on M17 agar plates comprising lactose. The agar plates were then incubated over night at 37 °C. Colonies were isolated using a colony picking robot (Genetix Q-pixil) and transferred to 384 well microtiter plates comprising M17 medium with 48 g/l lactose. The microtiter plates were then cultured for 24 hr and centrifuged at 4700 g for about 20 minutes. The supernatants were then diluted in water in a microtiter plate (45 µl water and 5 µl supernatant) and 5 µl of the diluted samples were transferred to microtiter plates comprising a mixture of glucose oxidase, peroxidase, 4-aminoantipyrine and phenol. The microtiter plates were shaken for 20 seconds at 1800 rpm and incubated for 15 min. Absorbance (505 nm) was measured and the concentration of glucose determined. Standards of glucose were used for quantification.

The culture medium of the wild type strains of the cultures 700, 702 and 706 showed accumulation of below 1 g/l glucose after 48 hr culturing in M17 medium comprising 48 g/l lactose. The standard deviation in the assay was approx. 5%. The experiment further showed that the analysis of approx. 10 000 samples is performed within about 4.5 hr. Screening of about 9600 mutants of the strain YF-L702 was then performed using the above screening method. A number of cultures accumulating glucose in the medium were determined. Fermentation of about 9000 mutants of the culture YF-LX700 was also performed.

### Example 5: Screening of mutants of a S. thermophilus strain.

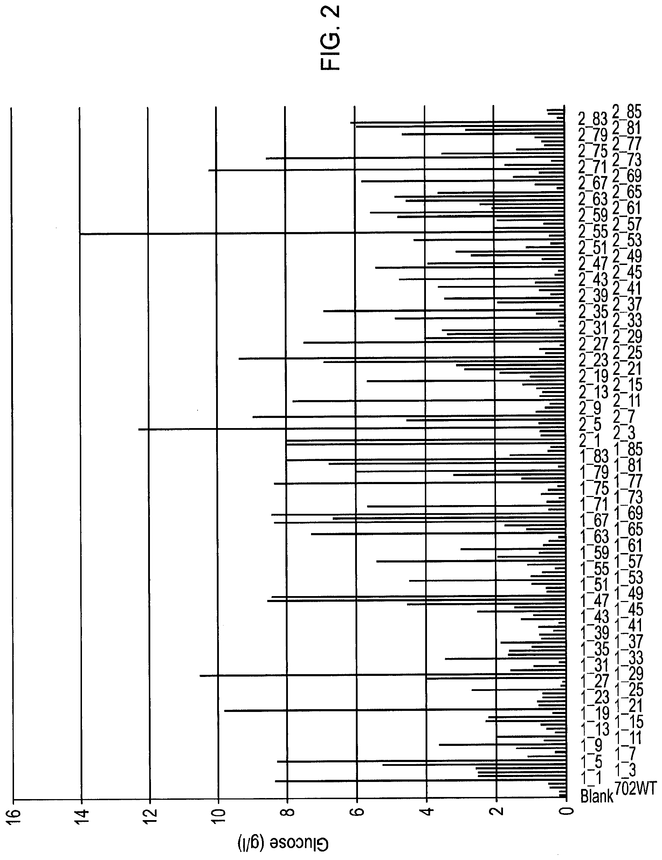

167 mutants obtained in example 4 were subjected to screening according to the present method. The selected mutants were transferred to 96 wells microtiter plates comprising M17 medium with 5 g/l lactose (150 µl in each well). Glycerol was added (18 vol%) and samples of the selected mutants were stored at - 80 °C. The mutants were then subjected to screening in 4 parallel in microtiter plates with 384 wells under the same conditions as in example 4. The results show that the method according to the present invention enables screening of a large number of strains within a reasonable time. The results furthermore show that the screening method provides for an efficient method to detect lactic acid bacteria strains having the ability to secrete more glucose into the medium compared with the parental strain, see figure 2.

The present inventors have also determined accumulation of glucose when culturing selected mutants identified by the present invention in milk (data not shown).

### Example 6: Fermentation of milk with mutant strains.

An isolate of *Lactobacillus delbruckii ssp. bulgaricus* was obtained from a commercial yoghurt culture after streaking on MRS (deMan, J. C., M. Rogosa, and M. E. Sharpe. 1960.. J. Bacteriol. 23:130). DNA sequencing of its 16S rRNA gene was used for identification. The isolate (about 107 CFU) was plated on a modified MRS medium containing 48 g/L lactose instead of 20 g/L glucose. The medium was supplemented with 20 mM 2-deoxyglucose. After incubation at 37 °C for two days, ten colonies were picked from the plate to another plate with the same medium. After another incubation at 37 °C for two days, colonies were transferred to and propagated in modified MRS medium. One of these isolates was named *Lactobacillus delbruckii ssp. bulgaricus* strain 9A. The strain was propagated in modified MRS medium at 37 °C. The 9A strain is deposited the 11^{th} July 2014 under accession number DSM29076.

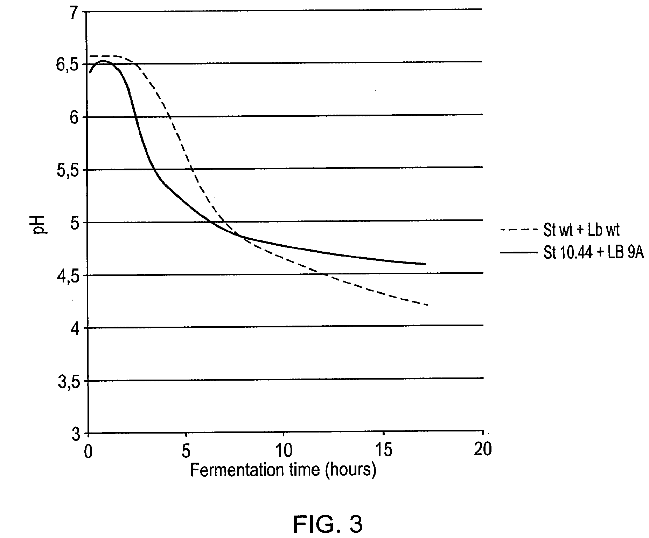

Milk was inoculated with a mixture of *S*. *thermophilus* strain 10.44 (isolated and identified with random mutagenesis and screening as shown in example 2, 4 and 5, and deposited the 11^{th} July 2014 under accession number DSM29077) and *Lactobacillus delbruckii subsp. bulgaricus* strain 9A and incubated at 42 °C. The pH was monitored continuously and recorded by a computer throughout the incubation period by the use of a pH meter with the electrode positioned in the milk. Before the inoculation in milk, *S*. *thermophilus* strain 10.44 had been grown overnight in M17 containing 48 g/L lactose and *Lactobacillus delbruckii subsp. bulgaricus* strain 9A in modified MRS. The inoculation level in milk was the same for both strains, each corresponding to OD₆₀₀ of 0.003. The pH of the milk during cultivation is shown in figure 3. The content of glucose, lactate, lactose and galactose at the end of cultivation were measured using HPLC. Samples were centrifuged at 16.800 rpm g for 5 minutes and filtered through 0.2 µm syringe filters (Gelman Sciences, Ann Arbor, USA) before HPLC-analysis. A Shimadzu chromatograph equipped with an autoinjector (SIL-9A, Shimadzu, Japan) and an Aminex HPX-87-H (BioRad Laboratories, USA) column operating at 45°C and a RI-detector (RID 6A, Shimadzu, Japan) was used. As eluent, 5 mM H₂SO₄ was used (0.6 ml min-1). Commercial standards were used for calibration. A concentration of 9.5 g glucose per liter was measured at the end of the fermentation period in milk inoculated with *S*. *thermophilus* strain 10.44 and *Lactobacillus delbruckii subsp. bulgaricus* strain 9A. Less than 1 gram glucose per liter was produced in cultures of mixtures of the parental wild type strains grown under the same conditions.

## Claims

1. A large scale screening method for identifying a glucose secreting lactic acid bacteria, wherein the method comprises the steps of:
i) subjecting lactic acid bacteria to mutagenizing conditions, wherein the mutagenizing condition consists in exposure of lactic acid bacteria to any conventional mutagens of physical (e.g. X-radiation, UV-radiation), or chemical (e.g. mutagens) origin;
ii) selecting the colonies obtained in step i) separately and transfer said colonies for growth in an array in growth media comprising lactose as the sole carbohydrate source;
iii) assaying the amount of glucose present in the culture supernatant after growth of step ii) by the use of:
a) glucose oxidase; peroxidase; and peroxidase signal substrate; or
b) glucokinase, hexokinase and glucose-6-phosphate dehydrogenase, ATP and NAD or NADP; or
c) glucose dehydrogenase and an electron acceptor;
wherein the amount of glucose secreted into the culture medium is proportional with the amount of signal substance obtained by the transformation of the peroxidase signal substrate in a) or the amount of NADH or NADPH produced in b), or the amount of reduced electron acceptor in c).

2. Method according to claim 1, wherein the lactic bacteria strains are sonicated after and optionally prior to step i).

3. Method according to claim 1, wherein the parenteral lactic acid strain is selected from the group consisting of strains of *Lactococcus spp*, *Streptococcus spp.* (e.g. *Streptococcus thermophilus), Lactobacillus spp.* (e.g. *Lactobacillus bulgaricus, Lactobacillus acidophilus*, *Lactobacillus subsp. casei), Leuconostoc spp., Pseudoleuconostoc spp, Pediococcus spp., Brevibacterium spp., Enterococcus spp, Propionibacteriusm spp.,* and *Bifidobacteriusm spp.*

4. Method according to claim 3, wherein the parenteral lactic acid strain is *S*. *thermophilus.*

5. Method according to claim 1, wherein the mutagen is selected from the group consisting of n-methyl-N'-nitro-N-nitroguanidine (NTG), and ethane methanesulphonate (EMS).

6. Method according to any of the above claims, wherein the screening is performed using glucose oxidase, peroxidase and a peroxidase signal substrate.

7. A method according to claim 1, for identifying a glucose secreting *S*. *thermophiles* strain, wherein the method comprises the steps of:
i) subjecting *S. thermophiles* bacteria to mutagenizing conditions, wherein the mutagenizing condition consists in exposure of lactic acid bacteria to any conventional mutagens of physical (e.g. X-radiation, UV-radiation), or chemical (e.g. mutagens) origin;
ii) selecting the colonies obtained in step i) separately and transfer said colonies for growth in an array in growth media comprising lactose as the sole carbohydrate source;
iii) assaying the amount of glucose present in the culture supernatants after growth of step ii) by the use of glucose oxidase; peroxidase; and a peroxidase signal substrate;
wherein the amount of glucose secreted into the culture medium is proportional with the amount of signal substance obtained by the transformation of the peroxidase signal substrate in iii).

8. Method according to any of the above claims, wherein the peroxidase substrate is selected from the group consisting of 4-amino-antipyrene, 4-amino-antipyrine, 2,2'-azino-bis(3-ethylbenzthiazoline-6-sulphonic acid) (ABTS), o-dianisidine, o-phenylenediamine (OPD), and 4-(2-pyridylazo)resorcinol, N-acetyl-3,7-dihydroxyphenoxazine (Amplex Red).

9. Method according to claim 7, wherein the peroxidase substrate is 4-amino-antipyrine.

10. Method according to any of the above claims, wherein the amount of signal substance or NAHD or NADPH or reduced electron acceptor formed in iii) (b) is measured photometrically.

11. Method according to claim 8, wherein the amount of signal substance formed is measured by spectrophotometry, luminescence or fluorescence.

12. Method according to claim 10, wherein the amount of NAHD is measured by spectrophotometry.

13. A glucose secreting lactic acid *S. thermophilus* strain selected from the group consisting of the *S. thermophilus* strain 2-83 deposited under the Budapest Treaty at DSMZ with the accession number DSM27535 and the lactic acid bacterial strain 10.44 deposited at DSMZ with the accession number DSM29077.

14. The use of a lactic acid bacterium strain according to claim 13 in the manufacturing of a fermented milk product.

15. Fermented dairy product comprising a glucose secreting bacterial strain according to claim 13.

16. The use of glucose oxidase, peroxidase, glucokinase, hexokinase, glucose-6-phosphate dehydrogenase and glucose dehydrogenase in a high through put screening method for identification of glucose secreting lactic acid bacteria, wherein said use involves exposing bacteria of a mutagenizing conditions, wherein the mutagenizing condition consists in exposure of lactic acid bacteria to any conventional mutagens of physical (e.g. X-radiation, UV-radiation), or chemical (e.g. mutagens) origin, and culturing bacteria growing on lactose as sole carbon source.

17. The use according to claim 16 of glucose oxidase, peroxidase, and glucokinase in a high through put screening method for identification of glucose secreting *S. thermophilus* strain.

18. Composition comprising at least one *S*. thermophilus strain according to claim 13 and at least one *Lactobacillus delbruckii spp. bulgaricus* strain.

19. Composition according to claim 18, comprising the *S. thermophilus* strain is the strain 10.44 (DSM29077) and the *Lactobacillus delbruckii spp. bulgaricus* strain 9A (DSM29076).

## Patentansprüche

1. Großmaßstäbliches Screeningverfahren zum Identifizieren von Glucose sekretierenden Milchsäurebakterien, wobei das Verfahren die folgenden Schritte umfasst:
i) Aussetzen von Milchsäurebakterien gegenüber einer mutagenisierenden Bedingung, wobei die mutagenisierende Bedingung aus einer Exposition von Milchsäurebakterien gegenüber beliebigen herkömmlichen Mutagenen physikalischen (z. B. Röntgenstrahlung, UV-Strahlung) oder chemischen (z. B. Mutagene) Ursprungs besteht;
ii) getrenntes Auswählen der in Schritt i) erhaltenen Kolonien und Überführen der Kolonien für ein Wachstum in einer Anordnung in Wachstumsmedien, umfassend Lactose als die einzige Kohlenhydratquelle;
iii) Untersuchen der Menge an Glucose, die in dem Kulturüberstand nach dem Wachstum von Schritt ii) vorliegt, unter Verwendung von Folgendem:
a) Glucoseoxidase; Peroxidase; und Peroxidasesignalsubstrat; oder
b) Glucokinase, Hexokinase und Glucose-6-phosphat-Dehydrogenase, ATP und NAD oder NADP; oder
c) Glucosedehydrogenase und einem Elektronenakzeptor;
wobei sich die Menge an Glucose, die in das Kulturmedium sekretiert wird, zu der Menge der Signalsubstanz, die durch die Transformation des Peroxidasesignalsubstrats in a) erhalten wird, oder der Menge an NADH oder NADPH, die in b) produziert wird, oder der Menge des reduzierten Elektronenakzeptors in c) proportional verhält.

2. Verfahren nach Anspruch 1, wobei die Milchsäurebakterienstämme nach und gegebenenfalls vor Schritt i) beschallt werden.

3. Verfahren nach Anspruch 1, wobei der parenterale Milchsäurestamm aus der Gruppe ausgewählt ist, bestehend aus Stämmen von *Lactococcus spp., Streptococcus spp.* (z. B. *Streptococcus thermophilus), Lactobacillus spp.* (z. B. *Lactobacillus bulgaricus, Lactobacillus acidophilus*, *Lactobacillus subsp. casei), Leuconostoc spp., Pseudoleuconostoc spp., Pediococcus spp., Brevibacterium spp., Enterococcus spp., Propionibacterium spp.* und *Bifidobacterium spp.*

4. Verfahren nach Anspruch 3, wobei der parenterale Milchsäurestamm *S. thermophilus* ist.

5. Verfahren nach Anspruch 1, wobei das Mutagen ausgewählt ist aus der Gruppe, bestehend aus n-Methyl-N'-nitro-N-nitroguanidin (NTG) und Ethanmethansulfonat (EMS).

6. Verfahren nach einem der vorhergehenden Ansprüche, wobei das Screening unter Verwendung von Glucoseoxidase, Peroxidase und einem Peroxidasesignalsubstrat durchgeführt wird.

7. Verfahren nach Anspruch 1 zum Identifizieren eines Glucose sekretierenden Stammes von *S. thermophiles,* wobei das Verfahren die folgenden Schritte umfasst:
i) Aussetzen von Bakterien von *S. thermophiles* gegenüber einer mutagenisierenden Bedingung, wobei die mutagenisierende Bedingung aus einer Exposition von Milchsäurebakterien gegenüber beliebigen herkömmlichen Mutagenen physikalischen (z. B. Röntgenstrahlung, UV-Strahlung) oder chemischen (z. B. Mutagene) Ursprungs besteht;
ii) getrenntes Auswählen der in Schritt i) erhaltenen Kolonien und Überführen der Kolonien für ein Wachstum in einer Anordnung in Wachstumsmedien, umfassend Lactose als die einzige Kohlenhydratquelle;
iii) Untersuchen der Menge an Glucose, die in den Kulturüberständen nach dem Wachstum von Schritt ii) vorliegt, unter Verwendung von Glucoseoxidase; Peroxidase; und einem Peroxidasesignalsubstrat;
wobei sich die Menge an Glucose, die in das Kulturmedium sekretiert wird, zu der Menge der Signalsubstanz, die durch die Transformation des Peroxidasesignalsubstrats in iii) erhalten wird, proportional verhält.

8. Verfahren nach einem der vorhergehenden Ansprüche, wobei das Peroxidasesubstrat ausgewählt ist aus der Gruppe, bestehend aus 4-Aminoantipyren, 4-Aminoantipyrin, 2,2'-Azino-bis(3-ethylbenzthiazolin-6-sulfonsäure) (ABTS), o-Dianisidin, o-Phenylendiamin (OPD) und 4-(2-Pyridylazo)resorcinol, N-Acetyl-3,7-dihydroxyphenoxazin (Amplex Red).

9. Verfahren nach Anspruch 7, wobei das Peroxidasesubstrat 4-Aminoantipyrin ist.

10. Verfahren nach einem der vorhergehenden Ansprüche, wobei die Menge der Signalsubstanz oder an NAHD oder NADPH oder des reduzierten Elektronenakzeptors, die in iii) (b) gebildet werden, photometrisch gemessen wird.

11. Verfahren nach Anspruch 8, wobei die Menge der gebildeten Signalsubstanz durch eine Spektrophotometrie, Lumineszenz oder Fluoreszenz gemessen wird.

12. Verfahren nach Anspruch 10, wobei die Menge an NAHD durch eine Spektrophotometrie gemessen wird.

13. Glucose sekretierender Milchsäurestamm von *S. thermophilus,* ausgewählt aus der Gruppe, bestehend aus dem Stamm von *S. thermophilus* 2-83, der gemäß dem Budapester Vertrag bei der DSMZ mit der Zugangsnummer DSM27535 hinterlegt ist und dem Milchsäurebakterienstamm 10.44, der bei der DSMZ mit der Zugangsnummer DSM29077 hinterlegt ist.

14. Verwendung eines Milchsäurebakterienstammes nach Anspruch 13 bei der Herstellung eines fermentierten Milchprodukts.

15. Fermentiertes Molkereiprodukt, umfassend einen Glucose sekretierenden Bakterienstamm nach Anspruch 13.

16. Verwendung von Glucoseoxidase, Peroxidase, Glucokinase, Hexokinase, Glucose-6-phosphat-Dehydrogenase und Glucosedehydrogenase in einem Hochdurchsatz-Screeningverfahren zur Identifizierung von Glucose sekretierenden Milchsäurebakterien, wobei die Verwendung das Exponieren von Bakterien gegenüber einer mutagenisierenden Bedingung, wobei die mutagenisierende Bedingung aus einer Exposition von Milchsäurebakterien gegenüber beliebigen herkömmlichen Mutagenen physikalischen (z. B. Röntgenstrahlung, UV Strahlung) oder chemischen (z. B. Mutagene) Ursprungs besteht, und das Kultivieren von Bakterien beinhaltet, die auf Lactose als einzige Kohlenstoffquelle wachsen.

17. Verwendung nach Anspruch 16 von Glucoseoxidase, Peroxidase und Glucokinase in einem Hochdurchsatz-Screeningverfahren zur Identifizierung von einem Glucose sekretierenden Stamm von *S. thermophilus.*

18. Zusammensetzung, umfassend mindestens einen Stamm von *S. thermophilus* nach Anspruch 13 und mindestens einen Stamm von *Lactobacillus delbrueckii spp. bulgaricus.*

19. Zusammensetzung nach Anspruch 18, umfassend, dass der Stamm von *S. thermophilus* der Stamm 10.44 (DSM29077) ist und der Stamm von *Lactobacillus delbrueckii spp. bulgaricus* 9A (DSM29076) ist.

## Revendications

1. Procédé de criblage à grande échelle pour identifier une bactérie lactique sécrétant du glucose, dans lequel le procédé comprend les étapes suivantes :
i) la soumission de bactéries lactiques à des conditions de mutagenèse, dans lequel la condition de mutagenèse consiste en une exposition de bactéries lactiques à des mutagènes classiques quelconques d'origine physique (par exemple, des rayons X, des rayons UV), ou chimique (par exemple, des mutagènes) ;
ii) la sélection des colonies obtenues dans l'étape i) séparément et le transfert desdites colonies pour une croissance en un réseau dans du milieu de croissance comprenant du lactose comme seule source de glucide ;
iii) l'analyse de la quantité de glucose présente dans le surnageant de la culture après la croissance de l'étape ii) par l'utilisation de :
a) glucose oxydase ; peroxydase ; et substrat signal de peroxydase ; ou
b) glucokinase, hexokinase et glucose-6-phosphate déshydrogénase, ATP et NAD ou NADP ; ou
c) glucose déshydrogénase et un accepteur d'électrons ;
dans lequel la quantité de glucose sécrété dans le milieu de culture est proportionnelle à la quantité de substance signal obtenue par la transformation du substrat signal de peroxydase en a) ou à la quantité de NADH ou de NADPH produit en b), ou à la quantité d'accepteur d'électrons réduit en c).

2. Procédé selon la revendication 1, dans lequel les souches de bactéries lactiques sont soniquées après et éventuellement avant l'étape i).

3. Procédé selon la revendication 1, dans lequel la souche de bactéries lactiques parentérales est choisie dans le groupe constitué par les souches de *Lactococcus spp*., *Streptococcus spp.* (par exemple, *Streptococcus thermophilus*), *Lactobacillus spp.* (par exemple, *Lactobacillus bulgaricus, Lactobacillus acidophilus*, *Lactobacillus subsp*. *casei*), *Leuconostoc spp., Pseudoleuconostoc spp., Pediococcus spp., Brevibacterium spp., Enterococcus spp., Propionibacteriusm spp.,* et *Bifidobacteriusm spp.*

4. Procédé selon la revendication 3, dans lequel la souche de bactérie lactique parentérale est *S. thermophilus.*

5. Procédé selon la revendication 1, dans lequel le mutagène est choisi dans le groupe constitué par la n-méthyl-N'-nitro-N-nitroguanidine (NTG), et le méthane-sulfonate d'éthane (EMS).

6. Procédé selon l'une quelconque des revendications ci-dessus, dans lequel le criblage est effectué en utilisant la glucose oxydase, la peroxydase et un substrat signal de peroxydase.

7. Procédé selon la revendication 1, pour identifier une souche de *S. thermophiles* sécrétant du glucose, dans lequel le procédé comprend les étapes suivantes :
i) la soumission de bactéries *S. thermophiles* à des conditions de mutagenèse, dans lequel la condition de mutagenèse consiste en une exposition des bactéries lactiques à des mutagènes classiques quelconques d'origine physique (par exemple, des rayons X, des rayons UV), ou chimique (par exemple, des mutagènes) ;
ii) la sélection des colonies obtenues dans l'étape i) séparément et le transfert desdites colonies pour une croissance en un réseau dans du milieu de croissance comprenant du lactose comme seule source de glucide ;
iii) l'analyse de la quantité de glucose présente dans les surnageants des cultures après la croissance de l'étape ii) par l'utilisation de glucose oxydase ; de peroxydase ; et d'un substrat signal de peroxydase ;
dans lequel la quantité de glucose sécrété dans le milieu de culture est proportionnelle à la quantité de substance signal obtenue par la transformation du substrat signal de peroxydase en iii).

8. Procédé selon l'une quelconque des revendications ci-dessus, dans lequel le substrat de peroxydase est choisi dans le groupe constitué par la 4-amino-antipyrène, 4-amino-antipyrine, 2,2'-azino-bis(acide 3-éthylbenzthiazoline-6-sulphonique) (ABTS), o-dianisidine, o-phénylènediamine (OPD), et 4-(2-pyridylazo)résorcinol, N-acétyl-3,7-dihydroxy-phénoxazine (Amplex Red).

9. Procédé selon la revendication 7, dans lequel le substrat de peroxydase est la 4-amino-antipyrine.

10. Procédé selon l'une quelconque des revendications ci-dessus, dans lequel la quantité de substance signal ou de NAHD ou de NADPH ou d'accepteur d'électrons réduit formé en iii) (b) est mesurée par photométrie.

11. Procédé selon la revendication 8, dans lequel la quantité de substance signal formée est mesurée par spectrophotométrie, luminescence ou fluorescence.

12. Procédé selon la revendication 10, dans lequel la quantité de NAHD est mesurée par spectrophotométrie.

13. Souche de bactérie lactique *S. thermophilus* sécrétant du glucose choisie dans le groupe constitué par la souche 2-83 de *S. thermophilus* déposée sous le Traité de Budapest à la DSMZ avec le numéro d'accession DSM27535 et la souche de bactérie lactique 10.44 déposée à la DSMZ avec le numéro d'accession DSM29077.

14. Utilisation d'une souche de bactérie lactique selon la revendication 13 dans la fabrication d'un produit laitier fermenté.

15. Produit laitier fermenté comprenant une souche bactérienne sécrétant du glucose selon la revendication 13.

16. Utilisation de glucose oxydase, de peroxydase, de glucokinase, d'hexokinase, de glucose-6-phosphate déshydrogénase et de glucose déshydrogénase dans un procédé de criblage à rendement élevé pour l'identification de bactéries lactiques sécrétant du glucose, dans laquelle ladite utilisation implique l'exposition des bactéries à des conditions de mutagenèse, dans laquelle la condition de mutagenèse consiste en une exposition des bactéries lactiques à des mutagènes classiques quelconques d'origine physique (par exemple, des rayons X, des rayons UV), ou chimique (par exemple, des mutagènes), et la culture des bactéries se développant sur du lactose comme seule source de carbone.

17. Utilisation selon la revendication 16 de glucose oxydase, de peroxydase, et de glucokinase dans un procédé de criblage à rendement élevé pour l'identification d'une souche de *S. thermophilus* sécrétant du glucose.

18. Composition comprenant au moins une souche de *S*. thermophilus selon la revendication 13 et au moins une souche de *Lactobacillus delbruckii spp. bulgaricus.*

19. Composition selon la revendication 18, comprenant la souche de *S. thermophilus* est la souche 10.44 (DSM29077) et *Lactobacillus delbruckii spp. bulgaricus* la souche 9A (DSM29076).
